# EUROPEAN PATENT APPLICATION

(11) **EP 3 725 257 A1**
(43) Date of publication of application: **21.10.2020**
(21) Application number: 19169476.9
(22) Date of filing: 16.04.2019
(51) Int. Cl.: A61B 90/00

(54) **LIGHT-POWERED LIGHT-EMITTING TISSUE MARKER**

(71) Applicant: Koninklijke Philips N.V., 5656 AG Eindhoven (NL)
(72) Inventor: MUELLER, Manfred, 5656 AE Eindhoven (NL); SABCZYNSKI, Jörg, 5656 AE Eindhoven (NL); KAHLERT, Joachim, 5656 AE Eindhoven (NL)
(74) Representative: de Haan, Poul Erik

(57) **Abstract**

Method and related system for marker-based navigation. A first image is capturing (S530) with a medical imaging apparatus (ENDO), whilst a light source (LS1) of an implanted marker device (MD) is in a low intensity state and a second light source (LS2) of the medical imaging apparatus (IA) is in a high intensity state, higher than the low intensity state of the marker. A second image is captured (S560) with the medical imaging apparatus (ENDO), whilst the light source (LS1) of the implanted marker device is in a high intensity state and the second light source (LS2) of the medical imaging apparatus (IA) is in a low intensity state, lower than the high intensity state of the marker. The two images may then be combined to obtain a combined image that represents the location of the marker at high signal-to-noise-ratio.

## Description

### FIELD OF THE INVENTION

The invention relates to a system for marker-based navigation, an implantable marker device for navigation support, a medical imaging device, a method for marker-based navigation, a computer program element, and a computer readable medium.

### BACKGROUND OF THE INVENTION

One of the main challenges during oncological surgery is the localization of a lesion. This may be an issue particular with small lesions and for minimally-invasive surgery.

A good example is resection of lung lesions using Video-Assisted-Thoracoscopic Surgery (VATS), in particular wedge resection. Wedge resection is the removal of tissue where the cuts do not necessarily follow borders of anatomical structures.

The lung will deflate during VATS which distorts the spatial relationship between points in and on the lung. The deflation typically makes it impossible to locate the lesion based on pre-operative imaging. VATS also means only indirect access to the lung through the chest wall via instruments and the lesion cannot be located by direct palpation. In addition, because wedge resections are not performed along visible anatomical structures or borders, anatomical features cannot be used as landmarks to guide the surgeon during the resection.

As a consequence, many surgeons prefer to do an anatomical resection (lobectomy or segmentectomy) even though this may result, in comparison to a wedge resection, in more healthy lung tissue being removed. The need for wedge resection gained more traction since the advent of lung cancer screening has led to a strong increase in the number of detected small, solitary, early stage lung lesions that need to be removed and that do not coincide with anatomical structures.

One way to deal with this issue is to pre-operatively place a marker inside or near the lesion. An overview of the use of such markers for lung tumors has been reported by J Keating et al, "Novel Methods of Intraoperative Localization and Margin Assessment of Pulmonary Nodules", Semin. Thoracic. Surg., vol. 28, Issue 1, pp. 127-136, 2016. The markers are typically placed in the inflated lung under CT guidance. Commonly used markers are radio-opaque coils or cylinders, small radioactive seeds or even liquid dyes.

Light emitting markers have also been proposed by Applicant in US 2017/0340406. However, even with such markers, it may still be challenging to successfully relocate such markers. Also, the powering for such marker may be difficult.

### SUMMARY OF THE INVENTION

There may therefore be a need for new approaches to address at least some of the above noted shortcomings.

The object of the present invention is solved by the subject matter of the independent claims where further embodiments are incorporated in the dependent claims. It should be noted that the following described aspects of the invention equally applies to the implantable/implanted marker device for navigation support, to the medical imaging device, to the method for marker-based navigation, to the computer program element, and to the computer readable medium.

According to a first aspect of the invention there is provided a system for marker-based navigation, comprising:
an implantable marker device having a light source, switchable into at least two intensity states, a low intensity state and a high intensity state;
a synchronizer logic; and
an imaging apparatus (also referred to herein as the "imager") with a second light source to provide incident light, the said second light source switchable into at least two intensity states, a low intensity state and a high intensity state;
wherein the light source of the marker device and the second light source are operable in synchrony by the synchronizer logic controlling the switching of the two light sources, the system thereby operable in two modes, in a beacon mode and in an exploration mode, so that, in exploration mode, the light source is switched into low intensity state and the second light source is switched into high intensity state, and so that in beacon mode, the light source is switched into high intensity state and the second light source is switched into low intensity state, the imaging apparatus controlled by the synchronizer logic, or by a user, to capture at least one respective image in the two modes.

In embodiments, the system includes an image processor configured to combine image information from the at least two images to form a combined image.

In embodiments, the image processor comprises a contrast enhancer to enhance contrast in the one or more images acquired in beacon mode.

In embodiments, the imaging apparatus includes a light filter component having its frequency response adjusted to a bandwidth of the light emittable by the light source of the marker device.

In embodiments, the image processor is configured to spatially register the image captured in beacon mode and the image captured in exploration mode. This registration functionality is beneficial if, after capturing the beacon image, a plurality of consecutive exploratory images is captured, and there is relative motion between the imager and the marker, such as caused by motion of the imager or by motion of the organ or anatomy that includes the marker.

In embodiments, the synchronizer logic comprises a light sensitive sensor, the light sensitive sensor operable to effect the switching into the two intensities of the first or the second light source, in dependence on incident light received from second or the first light source, respectively.

In embodiments, the light sensitive sensor is configured to facilitate i) the first light source to switch into high intensity state in response to the sensor sensing a light intensity below an intensity threshold and/or ii) the first light source to switch into low intensity state in response to the sensor sensing a light intensity above the threshold.

In embodiments, the light intensity sensed at the sensor is caused by the incident light from the second light source.

In embodiments, the low intensity state at light source of the marker or at the second light source of the imager is achieved by hard switching the respective light source off. The high intensity state of the light source of marker and/or of the second light source of the imager is achieved by simply switching the respective lights source on, to emit radiation at a certain intensity. The embodiments of switching off and on, as mainly considered herein, is by disconnecting or reconnecting power or by a using a shutter mechanism or other. The low and high intensity may be achieved through a dimmer mechanism. Preferably, the two light intensity states at the marker and imager are achieved both by a hard switching on and off, respectively, with a respective switch element. Alternatively, only the marker is switched on and off whilst the light source at the imager is switched into a low, no-zero, intensity state and the high intensity state, higher than the low intensity. This may be administered by (repeated) operation of a dimmer mechanism. Reducing the intensity to a low, non-zero, intensity level instead of a switching off the imager light source may allow picking up the marker light and still be able to image the surrounding anatomy to some extent. In the following, the system and method will be explained in terms of switch on and off events, but it will be understood that all of the below is of equal application if either one of the light sources is merely switched to a lower, non-zero, intensity state instead of being switched off.

In embodiments of the proposed system, one or more repetitive pulses from the marker can be synchronized with the light source of second light source of the imaging device, e.g., endoscope lighting, in order to enhance the signal-to-noise ratio of the light signal of the marker as captured in the imagery. In embodiments, whenever a light pulse is emitted from the light source of the marker, the other light source of the imaging device, e.g., an endoscope, is switched off. The resulting image (without illumination from the imaging device light source) may be contrast enhanced and/or optionally overlaid on an image acquired by the imaging device with the second light source switched on the image taken shortly before or after the light pulse issued from the marker. The imagery so obtained allows a user (that is, the person operating the imaging device and/or performing or facilitating the intervention) to quicker and more reliably locate the marker.

In this manner, the proposed marker may be used in particular for re-finding, during interventions or surgery, a location in cavities and/or in soft tissue with low or no ambient light. The location marked up by the previously implanted marker may be one of a lesion in organs, possibly situated in a body cavity or soft tissue or liquid being partly optical transparent. However, the marker device may also be used in other than medical contexts, such as in speleology or in thick forestry to assist a search crew returning to a previously marked site.

The proposed marker can be operated in a pulsating mode or continuous wave mode to facilitate its detection by the user. In a preferred embodiment, the marker's light source may be powered by a transducer that converts incoming or ambient light into energy which may be stored in an energy storage element, such as a battery.

The, in embodiments rechargeable, marker may be operated as follows. The marker is pre-operatively implanted (for example into the lung) into or near the lesion to be removed under image guidance. During the intervention, the inside of the patient is accessed and the anatomy of interest, e.g. an organ, is exposed to the light of a surgical lamp or the second light source of the endoscope. In case of a VATS procedure, the lung is also deflated. Light from the second light source of the imager may be strongly attenuated by the tissue surrounding the marker, but enough light energy is received to charge the marker. When the user needs to locate the tumor, he or she switches off the light at the endoscope and the marker will switch to beacon mode and emit light. Although the emitted light may also be attenuated. Typically, there is sufficient remaining light that may still be noticeable enough to help in locating the lesion. Ambient light in the operating theatre may be lowered to better discern the marker light. The marker may then be removed together with the lesion.

The synchronization logic may administer the switching of the light sources in a causally linked manner or in a non-causal manner.

In causally linked embodiments, the switching of the light source, in particular the switching on thereof, is caused by switching events in the light source of the imager. This can be implemented by the marker having the light sensitive sensor to switch the marker light source on or off.

In other embodiments, the causally linking synchronizer logic is configured to detect the switch operation directly, as through suitable interfaces and wireless communication arrangement that detect and communicate hard switches. A wired communication may also be envisaged, but this is less preferably in medical contexts but may still be used, preferably in non-medical context.

Non-causally linked embodiments of the synchronizer logic are also envisaged however, where the switching of the marker light source and the imaging device light source are not causally linked. This may be implemented by having the light sources pulsating by operation of two respective, autonomous, oscillator circuits with switching cycles out phase and with suitable pulse lengths so tuned so that the required synchronization is achieved. In particular, the light sources are switched so that the light source at the imager is off when the light source at the marker is on and vice versa.

In other words, the synchronizer logic may be implemented to effect, in one embodiment, causal or, in another embodiment, non-causal synchronization of the switching of the two light sources.

In causal synchronization, switching events at the marker are in response to switching event at the imager, or, switching events at the imager are in response to switching events at the marker.

In non-causal synchronization, the switching events at marker and imager are done autonomously but the switching events are so tuned that there are one or more instants where the maker light is off and imager light is on, and one or more instants where the marker is on and the image light is off. If there are more than two of such instants, these may be regularly (periodic) occurring or irregularly. In other words, at regular or irregular intervals one or more exploratory images and one or more beacon images can be acquired.

In another aspect, there is provided an implantable marker device for navigation support, comprising:
a light source configured to emit light;
a power source, configured to power the light source;
a light sensor configured to sense ambient light; and
a switch configured to switch the light source into at least two intensity states, a low intensity state and a high intensity state, in dependence on a light exposure at the light sensor.

In embodiments, the power source includes any one or more of: i) a transducer configured to convert incident light into electrical energy and ii) a battery. The transducer also to power the marker by light received during, the exploration mode, from the light source of the imager.

In embodiments, the device is wholly or partly embedded in a material, at least a portion of which is at least partly light transparent.

In embodiments, the device comprises one or more sensors configured to obtain one or more measurement values.

In embodiments, the device comprises a modulator configured to modulate the emitted light according to the one or more measurement values.

In embodiments, the measurement values relate to one or more physical or physiological quantities.

In embodiments, the emitted light is pulsed, continuous, or modulated in intensity.

In embodiments, the switch is configured to switch the light source into the high intensity state when an intensity of the incident light drops under an intensity threshold.

In embodiments, to facilitate implantability, the embedding material is formed as an encapsulation, such as convex hull. The marker may be monolithic. In particular, the embedding material may be monolithically enclosing the circuitry of the marker. The material may wholly or partly enclose the device. In other embodiments, the encapsulation is not necessarily convex, and may include protrusions that serve as anchor portions for in- or on-tissue anchoring the device. The device is preferably biocompatible. In embodiments, the embedding material is biocompatible, such as glass or other.

In one embodiment, the device has a shape and size so as to be administrable through a medical needle or a catheter or other delivery device.

In one embodiment, the marker device may include a microcontroller, such as FPGA or an ASIC or further electronic circuitry coupled to one or more of the measurement sensors to facilitate measurement operations in relation to physical or physiological properties whilst implanted. Instead of being powered by light, the marker may include an on-board battery that may be non-rechargeable or, preferably, chargeable. In this case, no transducer is required.

In one embodiment, the synchronization logic may be arranged as circuity such as a microcontroller or chip, such as an FPGA or ASIC. The synchronization logic may be integrated into the imaging apparatus, or into the marker. Alternatively, some or all of the synchronization logic circuitry is arranged in an external processing unit, arranged remotely from the device and/or imaging apparatus.

In another aspect, there is provided a medical imaging device, comprising an image sensor to capture an image and a light source to provide ambient light for the light sensor, and a synchronizer logic operable to cause the light source being switched into at least two intensity states, a low intensity state and a high intensity state, whilst capturing images, the said switching on and off being set at a preset frequency or wherein the switching between the two intensities is in response to an external switching signal received by the synchronizer logic through an interface of the device.

The synchronization logic of the imager is configured so that the light source of the imager operates in synchrony with an external light source, in particular the light source or the implanted/implantable marker device. The synchronizer logic is adjusted so that there are one or more instants or periods where the light source of marker is on and the light source of the imager is off and that there are one or more instants or periods where the light source of the marker is off and the light source of the imager is on, so that on or more pairs of beacon and exploratory images can be captured.

In one embodiment, the interface of the device includes a light sensor similar to the one described above in relation to the marker. In embodiments, the light sensor is at the imager instead of at/in the maker, and in this embodiment it is the marker that is controlling the switching of light source of the imager.

In one embodiment, the preset frequency of the light source of the imaging device is set in dependence on a pulsed frequency of the light source of the marker-device. The present frequency and/or pulse length may be adjusted by the user. The frequency refers to a pulsed mode of the light source and relates to the time between two such pulses. The light of the illumination source used in the imaging device is preferably white light.

The switching signal may originate external of the imaging device and is preferably received from the implanted marker. Alternatively, the switching signal of the light source at the imaging apparatus is issued by the user of the imager, by operation of a suitable manual light switch.

In another aspect there is provided a method for adjusting a switch threshold of the bio-compatible, implantable marker device. The method comprises:
receiving a specification of an operational light intensity of a second light source associated with an imaging apparatus;
setting the threshold as a function of the said specification.

This is useful in embodiments where the marker is light powered. By setting the threshold low enough, it can be avoided that the marker switches unintentionally on, and thus possibly wasting energy, when the light of the imager is left on but is merely directed away from the location of the marker, such as when the user examines the surroundings.

Using a light powered marker device as proposed herein in a preferred embodiment, allows providing a small, compact marker device that is safe to use, has a long shelf life and is unlikely to interfere with normal workflow.

The proposed system and marker allows obviating issues that may arise when electro-magnetic waves in the Gigahertz frequency range are used such as in Applicant's US 2017/0340406. In those systems, the electro-magnetic radiation does not penetrate deeply enough into biological tissue. In addition, bulky antennas for both sending and receiving may then be required increasing the overall product footprint of the marker. There are trade-offs here: by using lower frequencies, higher penetration depths are feasible, but at the cost of larger antennas. Directional antennas for sending can concentrate the field energy at the receiving antenna. Unfortunately, these directional antennas are even larger.

Alternatively, the light-emitting marker may be powered by an on-board integrated battery which stores electrical energy. Although such batteries are envisaged herein in embodiments, physically small batteries typically have a limited capacity and a limited shelf life. Rechargeable batteries or capacitors may be used and charged before deployment of the marker implementation, although this step may require sterilization and may hence lead to a cumbersome workflow in the operating theatre.

The light powered marker device proposed herein sidesteps all of these concerns.

In another aspect there is provided a method for marker-based navigation, comprising the steps of:
capturing a first image with a, preferably medical, imaging apparatus, whilst a light source of an implanted marker device is in a low intensity state and a second light source of the imaging apparatus is in a high intensity state, higher than the low intensity state of the marker; and
capturing a second image with the imaging apparatus, whilst the light source of the implanted marker device is in a high intensity state and the second light source of the imaging apparatus is in a low intensity state, lower than the high intensity state of the marker. In embodiments the switching of the marker light source is in response to the switching of the light source of the imaging apparatus, such as in causal synchronization.

In embodiments, the method further comprises: combining the two images and, optionally, displaying the combined image on a display device. In the combined image, a signal-to-noise contrast is improved in relation to the marker location. The marker location may hence by found more reliably by the user, based in the imagery. Alternatively, the two images are displayed, either simultaneously on the same or on different display devices, or in sequence.

In another aspect, there is provided a computer program element, which, when being executed by at least one processing unit, is adapted to cause the processing unit to perform one or more steps of the method.

In another aspect, there is provided a computer readable medium having stored thereon the program element.

### BRIEF DESCRIPTION OF THE DRAWINGS

Exemplary embodiments of the invention will now be described with reference to the following drawings, which are not to scale, wherein:
Figure 1 shows a block diagram of a system for marker based navigation;
Figure 2 shows switching cycles of two synchronized light sources;
Figure 3 shows a block diagram of an implantable marker device;
Figure 4 shows circuitry of a marker device; and
Figure 5 shows a method for marker based navigation.

### DETAILED DESCRIPTION OF EMBODIMENTS

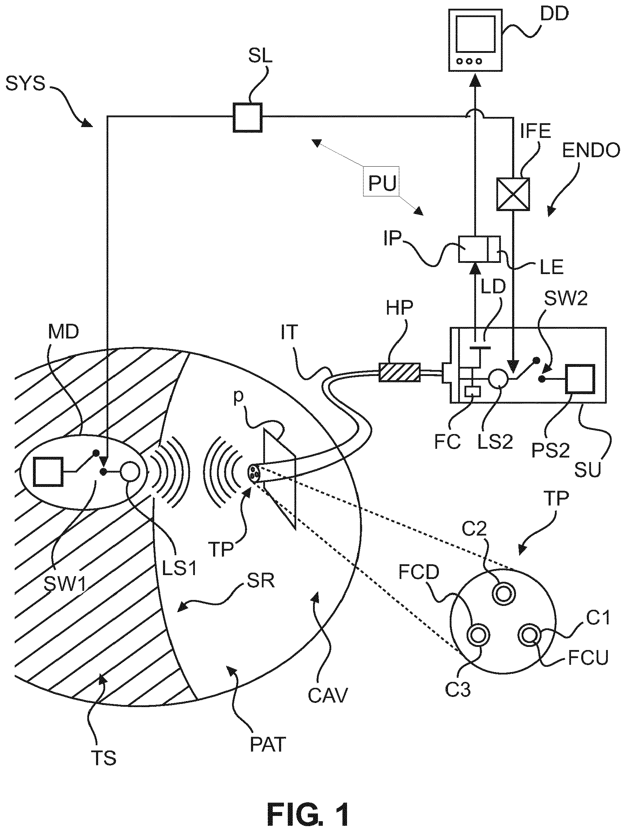

With reference to Figure 1, this shows a schematic block diagram of a system SYS for marker assisted navigation as may be used in medical interventions such as minimally invasive surgery (MIS) interventions, in-situ inspections or similar. In particular, the system SYS preferably supports imaging during such interventions.

Medical applications, in particular MIS applications, include lung interventions such as VATS. VATS interventions include the removal of lesions from lung tissue, for example wedge resections and similar. Other MIS medical applications may include inspection of intestines, urethra, esophagus, liver, and other organs or groups thereof.

Although medical applications are mainly envisaged herein, non-medical applications are also envisaged, such as navigation in plumbing systems, in cave systems or in other difficult to access environments where a certain target needs to be located.

The system SYS includes broadly a marker device MD which is implantable in human or animal patient PAT. The marker device includes a light source LS1. The marker further includes a switch SW1 to switch the light source LS1 on and off, the purpose of which will be explained more fully below. The system SYS further includes a medical imaging device ENDO, such as an endoscope. Although main reference will be made in the following to endoscopic imaging, other imaging modalities are also envisaged herein, such as a digital camera, a fiberscope, a rhinoscope, an arthroscope, a laparoscope, a surgical microscope, a telescope, and other. Capsule endoscopes are also envisaged, that are administered into the patient as whole.

A part of the endoscope ENDO is introducible into the patient to acquire images to provide visual support for navigation and intervention. A light source LS2 of the endoscope ensures sufficient exposure when acquiring images from inside the patient PAT. The marker device MD was implanted into the patient PAT close to a lesion, in a procedure prior to the endoscope supported intervention to be described herein.

Very broadly, in the system SYS as envisaged herein, the light source LS2 of the endoscope ENDO and the light source LS1 of the marker device MD act together to assist the user in navigating to the marker location and hence to the lesion. The two light sources are switched in synchrony to acquire imagery that reveals the location of the marker MD and hence of the lesion. The imagery may be processed by an image processor IP to better visualize the marker location. The image processor IP may facilitate displaying of the imagery on a display device DD to yet better support the user during the intervention.

Before explaining operation of the system SYS in more detail, we refer first to the imaging device ENDO to describe some of its components.

The endoscopic imaging device ENDO allows the user to "see" inside the patient. The imaging apparatus ENDO operates preferably using non-ionizing radiation, such as visible light. The imaging device includes a supply unit SU, optionally a hand-piece HP and an insertion tube IT.

The supply unit SU may include the light source LS2. The light source LS2 may be powered by an onboard power source PS2 in the supply unit SU, or it may be connectible to an external power source. The light source LS2 is switchable on and off by a switching element SW2. Specifically, the switch SW2 may operate to connect or reconnect the light Source LS2 to the power source PS2.

The insertion tube IT may be connected to the hand piece HP. The hand piece allows operation by the user of the endoscope ENDO. The insertion tube is for at least partial insertion into the patient PAT. The insertion tube IT has sufficient stiffness so that it can be urged into the patient and may be advanced through the patient. The insertion tube IT may include a metallic mesh coated by a polymer. The insertion tube terminates in a distal tip portion TP.

In embodiments, there the imaging apparatus may include a steering mechanism. In particular, a distal portion of the tube TP including the tip TP forms a bendable section. The bendable section is articulated by a series of inter-joined segments. A plurality of wires, such 2, 3, 4 or more are attached to the tip at different anchor points. The wires run parallel the bending section, inside the tube IT and the full length thereof, from tip TP to hand piece HP. In the hand piece, the wires couple into an actuator such as a mechanical chain-sprocket arrangement or other. The user can tension the wires by way of the actuator, to force the tip to curl and bend into different spatial directions as desired, depending on which wire is tensioned most. The actuator responds to knobs or levers at the hand piece operable by the user. The bendable section thus allows the user to navigate and negotiate obstacles.

To the lower right in Figure 1, there is shown a cross section of tube IT at the tip TP through a plane p. Inside the insertion tube there are a number of channels, such C1 - C3. One channel, the working channel C1, allows introduction of medical tools to perform the intervention or the removal of re-sected tissue by application of a vacuum for instance. The other two channels C2 and C3 include respective fiber cables FCD, FCU which run the full length of the insertion tube IT from the distal tip TP portion back into the supply unit SU. The fiber cables may terminate at tip portion TP in respective lenses (not shown).

The light source LS2 is to provide illumination, in particular so that imagery at sufficient exposure can be acquired. To this end, and in some, but not all embodiments, the light source LS2 is optically coupled to one of the optical fibers FCD to send light down the FCD cable, to the tip portion TP and into the surroundings to provide illumination thereof. Light reflects off structures encountered by the outgoing light and is captured by the second fiber cable FCU. The captured light travels up that second fiber cable FCU which also runs the full length of the tube IT, back to the supply unit. The second fiber cable FCU, at its proximal end, is optically coupled to a camera system that includes the image sensor LD to capture imagery. Acquisition Circuity (not shown) allows capturing imagery at a suitable frame rate, such as 20-30 fps or other.

The imagery so captured may then be processed by the image processor IP, on which more further below. The imagery is processed into a video feed which can be displayed on the display device DD in grey scale or in color or in any other suitable rendering. Still imagery may also be acquired and presented for display, if required. There may also be an optical eye piece to observe the inside of the patient instead of or in addition to the camera system described.

It will be understood that the described endoscopic system ENDO is merely according to one non-limiting embodiment. Numerous other endoscopic imaging system in a multitude of variations are also envisaged herein and may be used in the context of the described system SYS. For instance, the camera system may be situated outside the supply unit SU. Not all endoscopic imagers may have a dedicated hand piece HP as described. The tip portion may be structured differently.

Not all endoscopes may necessarily have an articulated bending section as described and the number of channels may be different from what was described above.

Also, the optical fibers UFC, DFC are not necessarily required in all embodiments. For example, in alterative embodiments, the camera system including the image sensor TD (such as a CCD) and one or more lenses may be arranged at the tip TP. In addition or instead, he the light source(s) LS2, such as one or more LEDs, might also be arranged at the tip TP.

Furthermore, surgical endoscopes such as laparoscopces, thoracoscopes, arthroscopes, etc., may not necessarily have a working channel. In surgical procedures, instruments are usually introduced into the patient via through additional access points.

Endoscopic imaging is not necessarily required herein in all embodiments. Specifically, in embodiment as surgical instrument where the insertion tube is a needle or trocar. In this or similar cases, the insertion tube is stiff, does not include a bendable section, and there is no steering mechanism. The camera system TD and light source LS2 are again both arranged at the tip of this stiff insertion tube.

As indicated, the system SYS allows the user to localize, within a patient PAT, a site of interest, such as lesion, in proximity of which the marker MD has been implanted in an earlier procedure. Specially, the marker MD may have been introduced into the patient through a medical needle, a catheter, a working channel of an endoscope, or other delivery device, in a preparatory phase before the endoscope or other imaging device supported intervention. The marker may be delivered without any endoscope, e.g. transthoracically with a needle under CT guidance or similar. At delivery, the marker MD may be plunged into tissue TS, close to where the lesion is located. More than one marker may be implanted so as to circumscribe the location of the lesion. The implanting of the marker may have been carried during X-ray imaging guidance, such as CT. Later, the site may need to be revisited in a different procedure to carry out the intervention, and it is these and later procedures that are the main focus herein. Instead of the described endoscopic imaging device ENDO, other imaging devices may be used in such procedures to navigate to the lesion site.

Because some imaging devices, such as some endoscopic imaging devices ENDO, use non-ionizing radiation for imaging, a line of sight through air is required to image structures of interest. It may be the case that the lesion, and hence the marker MD planted close to it, is located inside the tissue or organ, behind a tissue/organ outer surface SR. So even if the endoscope ENDO is introduced into a cavity CAV bounded by the surface SR, there may still be no line of sight between the endoscope and the marker device MD because of occlusion by intervening tissue TS.

To address this, the light source LS1 of the marker device MD is configured to emit light of sufficient intensity so the light is capable to penetrate through the intervening tissue for a reasonable distance, and possibly into the cavity CAV (if any), to be detectable, albeit attenuated, by the image sensor LD of the endoscopic imager ENDO.

It is envisaged herein to switch on and off, in synchrony, the two light sources LS1, LS2 of the marker device MD and of the endoscope ENDO, respectively. This allows the user to better localize the marker device possibly buried in surrounding tissue with no direct line of sight. More specifically, it is envisaged herein that the light source of the marker MD is switched off when the light source LS2 of the imaging device ENDO is switched on, and, vice versa, that the light source LS1 of the marker MD is switched on when light source LS2 of the endoscope is switched off. In addition, imaging by the imager ENDO is also synchronized so that, in either of the two synchronized states, one or more images are acquired by the imager ENDO.

As the distal tip portion TP of the endoscope ENDO resides during the intervention inside the patient there is very low ambient light, or even next to complete darkness. Imaging whilst the illuminating source LS2 of the endoscope is off will therefore lead to dark images with flat signal distribution, expect for an isolated light signal that is recorded as a local bright "splodge" because the marker MD's light source is on whilst the endoscope's light LS2 is off. The one (or more) dark image with the isolated bright signal may be referred to herein as a "beacon image". The beacon image may thus provide the user with a clear clue as to the location, or at least a direction, where the marker device MD is located. Once one or more of such beacon images, with the localization information encoded, are acquired, the light source of the endoscope ENDO is switched on again but now the light source of the marker MD is switched off. The endoscope ENDO continues to acquire one or more images, but this time the images, which may be referred to herein as "exploratory images", are now fully illuminated to reveal of anatomic structures of surroundings that were missing in the beacon images. However, in the exploratory images it is now the location of the marker that is missing.

The synchronization of switching the two light sources LS1 and LS2 in this manner during imaging hence leads to two intertwined streams of images, the fully illuminated ones and the dark images. The imagery from the two streams may be displayed in alternate fashion. It is proposed herein to process by the image processor IP the images from the two streams in combination to obtain the complete information, namely the location of the marker and the anatomical information.

By selectively switching the two light sources on/off in synchrony, the system SYS overall operates in two modes, in beacon mode and in an exploration mode. When in beacon mode, only the marker device light source LS1 is on whilst the endoscope light source LS2 is off and the imager ENDO captures the beacon or dark images. When in exploration mode, the roles are reversed, in that the marker MD light is now off whilst the light source LS2 of the endoscope is on to capture the exploratory, fully illuminated images.

Oscillation between those two modes, that is the synchronization of the switching operations at the marker MD and endoscope ENDO, may be administered and controlled by a synchronizer logic SL. At least a part of the synchronizer logic SL may be located external to the endoscope and the marker device, implemented on one or more data processing units, coupled through wired or wireless communication with remaining parts of the synchronizer logic SL. Alternatively, the synchronizer logic may be partly or fully integrated into the marker device or partly or fully integrated into the endoscope. One part of the synchronizer logic SL may be integrated into the endoscope and another part in the marker MD, as required.

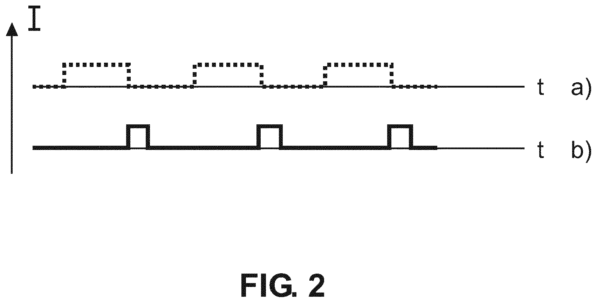

The synchronizer logic SL ensures out-off phase switching cycles or pulsing frequencies of the two light sources LS1 and LS2. A pattern of switching cycles of the light sources as envisaged herein is shown in diagrams a) and b) in Figure 2. Diagram a) pertains to the imager light source LS2. Diagram b) pertains to the marker MD light source LS1. The two diagrams show respective light intensity *I*-versus-time-*t* curves for light sources LS1 and LS2. Zero intensity period (referred to herein as "switch off period") corresponds to a period where the respective light source LS1, LS2 is off. The non-zero intensity periods (referred to herein as "switch on period"), that is the light pulse length, corresponds to a period during which the respective light source LS1, LS2 is on. The flanks mark the switching events, either from "on" to "off' or from "off to "on". Specifically, curve a) represents the switching cycle of the endoscope light LS2 and curve b) represents the switching cycle of the marker device source LS1. In particular, a switch-on period of the marker device in b) falls within a switch-off period of the endoscope cycle and, vice versa, the switch on period of the endoscope falls within the switch off period of the marker device.

Preferably the switch on period of the marker light source is shorter than the switch on period of the endoscope, such as in circumstances, where fewer beacon images are acquired than exploratory images. In in extreme example, the switch on period is only for a single beacon image to be acquired so the switch on period of LS1 is quasi-instantaneous, with a spiked pulse and with a relatively long switch off period. In contrast, the switch on period of the endoscope light SL2 is longer, allowing the acquisition of a plurality of exploratory images.

In embodiments, a switch off period of one of the light sources coincides with the switch on period of the other light source.

The frequency of the switching cycle of the marker device light source may be the same or may differ from the frequency of the switching cycle of the endoscope light source.

The cycles may not to be static, but one or both may be variable, adjustable by the user. In one embodiment, one setting may correspond to the images of the two streams strictly alternating, every one beacon image followed by one single exploratory image which is then followed by one single beacon image and so forth. This cycle setting may be useful if the user has difficulties locating the marker. However once found, the marker light frequency can be decreased. Generally, however the cycles are such that one or more beacon images are followed by a run of exploratory images. Furthermore, cycles may not necessarily be regular. In fact, in some embodiments, the cycle of the imager light source LS2 may be irregular, the light LS2 being switched on and off at the user's leisure.

Preferably, some or each switch off event at the imager light source LS2 causes a switch on event at the maker device light SL1. Preferably, a switch on event at the imager light source LS2 causes the light source LS1 of the marker to switch off, although this is not necessarily required in all embodiments, as the light source LS1 in the marker may switch off, for instance on its own, before the imager light source LS2 switched on as shown in Figure 2.

When in beacon mode, the light source LS1 of the marker may give of light continuously for a preset period of time or the light is given off in pulsation, as a series of pulses at a frequency for a preset period of time, or until the imager light source LS2 is switched on again.

It is not necessarily the case in all embodiments that the imager's illumination source LS2 switches on at each and every instant when the marker light source is off, such as during pulsation of the marker light source LS 1 as triggered by an earlier switch off event at the imager's illumination source LS2.

In general, the synchronizer logic SL may be implemented as causally linked or where there is no causal link between the two switching of switches SW1, SW2. These embodiments may be referred to herein as a causally linking synchronizer logic SL or a non-causally linking synchronizer logic SL.

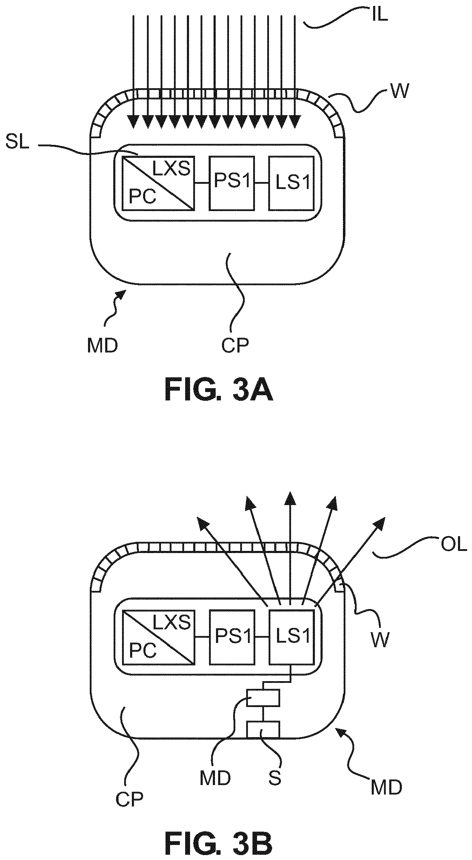

Referring now first to the causally linked synchronizer logic SL, this may be implemented partly at the marker device as will now be explained with reference to Figure 3. The synchronizer logic SL may implement a cycle pattern as shown in Figure 2.

Specifically, Figure 3 shows a schematic block diagram of a marker device MD arrangement according to one embodiment. Figure 3A shows the marker device illuminated by ambient light, for instance incoming IL light that is coming from the light source LS2 of the imaging apparatus ENDO. Figure 3B shows a state where there is no incoming illumination IL, but instead it is now the marker device's own light source LS1 that is activated and outgoing light OL issues forth from the marker device.

The circuitry of the marker device includes the above mentioned light source LS1 and an onboard power source PS1 that powers the light source LS1. The power source may be a re-chargeable battery, a non-rechargeable battery or a capacitator or other. The switch SW1 is operable to connect and reconnect the light source LS1 to the power source PS1. For ease of representation, in Figure 3 the marker MD's switch SW1 is not shown.

The light source LS1 may be arranged as a light emitting diode (LED), or similar. In embodiments, red or NIR radiation is preferred in case of LEDs as this ensures sufficient tissue penetration depth. Alternative, a high power (white) light bulb or a stroboscope light is used.

In a preferred embodiment, the circuitry of the marker device MD further includes a transducer PC. The transducer PC may be arranged as photo cell or photo diode. The transducer PC is capable of converting incoming light IL, such as from the endoscope light source LS2, into electrical energy so as to power the light source LS1 of the marker MD.

The transducer PC may either supply directly the light source with electrical energy but preferably there is a buffer storage element, such as the capacitator or rechargeable battery PS mentioned above, that stores the energy converted by the transducer PC and the light source LS1 draws the electrical energy from the power storage element PS.

Some or all of the mentioned components, that is, the light source LS1, the power source PS and the converter PC, if any, are preferably arranged on a single semi-conductor chip or a PCB although this may not be necessarily so in all embodiments where some or all components are distributed across two or more semi-conductor chips.

In a preferred embodiment, but not in all embodiments, the marker device MD further includes a light sensitive sensor LXS. The light sensitive sensor forms part of the causally linking synchronizer logic SL. The light sensor LXS facilitates switching on the light source LS1, if no ambient light is sensed by the sensor LXS, for instance, in response to switching off the endoscope light source LS2. In addition, the light sensitive sensor LXS facilitates switching off light source LS1 in response to sensing incoming light, such as light form the endoscope source LS2. The light sensitive sensor LXS and the transducer PC, if any, may be arranged in a single unit such as a photo diode or photo cell, or may be arranged discretely and separately as different components. In simpler embodiments, there is no light sensor LXS and the power source is a rechargeable battery or, less preferred, a non-rechargeable battery.

The light sensitive sensor establishes a causal link between the switching events at the endoscope and the marker MD. The switching at the marker responds to events at the endoscope. The switching events and imaging at the endoscope may be purely user controlled and triggered by the user operating a button or other actuator. Alternatively, there is an automatic switching circuitry in the imager ENDO, such as a multiplexer to toggle/switch between different predefined modes or a multi-vibrator or other which the user can set up by specifying through a user interface the switching cycle. The source LS2 is then repeatedly switched on and off at the specified frequency and period lengths and whilst the imaging continues. The marker MD, thanks to sensor LXS will then respond accordingly as per pattern as in Fig 2 b). In this and similar embodiments, the imager may include an interface IFE to receive the switching command from the switching circuitry.

If the imaging device ENDO is of the endoscope-type, the insertion tube IT may include a further channel (not shown in Figure 1) dedicated for delivering light from a different, second light source of the imager. For example, a non-visible near infrared (NIR) light channel may be used to power up the marker so as not to interfere with light through another channel to provide the visible illumination light from source LS2 for imaging purposes. There may also be an additional working channel (not shown), in addition to the working channel C1, suitably dimensioned with a lumen for delivery or removal of the marker.

Although in some embodiments the light source LS2 is integrated in the medical imaging device ENDO, this is not necessarily so in all embodiments, where the illumination source is separate from the imager imaging device ENDO. The illumination source may be introduced into the patient through a different delivery tool.

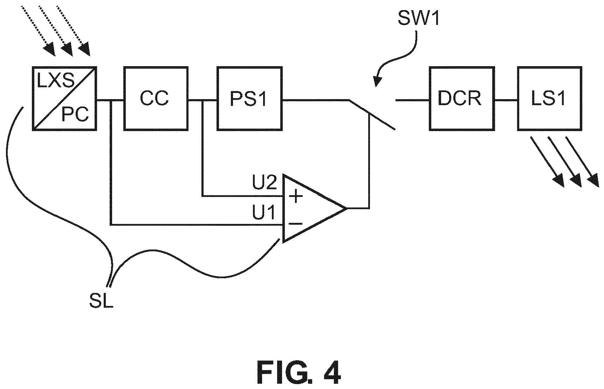

Figure 4 shows further details of the marker MD circuitry configured to implement the marker device MD as per Fig 3 according to one embodiment. The marker device MD includes the light sensitive sensor LXS and, optionally, the transducer PC. The synchronizer logic SL comprises the light sensitive sensor LXS and logic circuitry such as a flip-flop FF as shown in Figure 4. Incident light, such as coming from the endoscope light source LS2, is shown in dashed arrows. When light is received, the transducer converts this into energy and stores the same in the power source PS such as a battery through charging circuit CC. The circuitry is operable in charging mode and in a mode that give rise to the earlier mentioned beacon mode.

More specifically, when incident light IL is sensed at the sensor LXS and if a transducer PC is present, the power source PS is charged by transducer PC and the light source LS1 is switched off, with no power supplied to the light source LS1. However, when no light is received at the sensor LXS, the transducer is no longer charging and the device MD switches into beacon mode. Now, power source PS1 does provide power to the light source LS1 to release light (shown in solid arrows).

In more detail, and with continued reference to Figure 4, the marker's illumination source LS1 can be operated in a pulsed on/off mode. During the switch on period, the sensor LXS captures photons and charges the energy storage element PS1, such as capacitor. When the illumination IL is discontinued, the marker device automatically switches into beacon mode. In this mode, the storage element PS 1 now powers the light source LS1 and the light source emits a light signal, either pulsed or in continuous wave mode (cw). When the sensor LXS receives photons, it is in charging mode. In the charging mode, the output voltage at the sensor LXS is higher than the voltage at the energy storing element PS1. When the illuminating beacon light source PS1 is switched off, the output voltage at the sensor LXS drops and will be lower than the voltage supplied by energy storing element. These oscillating voltages U1, U2, can be processed by a flip-flop trigger circuitry FF as part of the synchronizer logic SL to trigger switch SW1. Once so triggered, the stored energy from the storing element PS1 can flow as an electrical current through the light source LS1 which causes emitting of light. The voltage drawn from the power source PS1 in beacon mode power may be provided by an LED driver circuit DCR which enables the light source LS1 to illuminate.

In embodiments when the power storage PS1 of the marker MD is recharged by light captured at the light sensor LXS or other charging arrangement, the switch off period preferably is at least as long as the recharge period as shown in Fig 2. However, it is in particular in non-causally linked embodiments of the logic SL where there is no sensor LXS required, and one or both light sources are powered by batteries or by wired or wireless connection to an external power supply.

The pulse length of the emitted light pulse, that is the length of the switch on period, may depend on the amount of energy stored in the capacitor. If the sensor LXS is in close vicinity to the illuminating source LS2 of the imager, more energy is stored and the maker light source LS1, the "beacon", may emit light for a longer period of time with a longer switch on period. The switch on period of the marker may hence be used as a "surrogate" measure to determine the distance from the illuminating source LS2 and the beacon LS1. In other words, in embodiments, a processing unit PU, either integrated in the imager ENDO or external thereto, can convert the switch on period as measured through a series of beacon images into a distance. The distance so computed may be displayed on the display device, overlaid on the current exploratory image.

In some embodiments of Figs 3,4, it is the user who switches on/off, with a manual switch, the light source LS2 at the imager ENDO, as required, preferably when requesting imaging in exploratory or beacon mode. When switching off the light source LS2 at the endoscope, and as a direct response thereto, the light from illumination source LS2 is now received at the light sensor of LXS of the marker device, and the synchronizer logic SL then operates the onboard switch SW1 in the marker device to switch on the light source of the marker device as described above. It will be understood that in this embodiment, a light intensity at which the sensor LXS causes the switching off of the marker source LS1 may need to be adjusted, for instance in a calibration setup, to ensure that his threshold is not set too high. For instance, the maker light source LS1 should preferably be not merely switch on when the illumination source LS2 is pointed away from a direction of the marker as the user examines the surroundings.

A converse arrangement to the one of Figs 2,3 is also envisaged, where the light sensor LXS is instead integrated into the endoscope. In embodiments, the sensor LXS is arranged on the insertion tube at its bending section or at the insertion portion of a delivery device such as a needle, etc. Specifically, the sensor LXS may be integrated in the tip TP portion. In this embodiment it is now the marker device that controls the switching off of the endoscope light source LS2 as opposed to the above mentioned embodiments Figs 3,4, where it is the endoscope light that controls the switching off of the light source at the marker device. In this converse embodiment, the switching signal interface IFE may include the light sensitive sensor in order to receive the switching on/off commands, that is, the light signals from the marker light source LS1, to switch the switch SW2 accordingly. This converse arrangement may be useful to ascertain the optical transparency of the surrounding tissue.

The embodiments in Figs 3,4 described above, where the marker device includes the light sensor LXS to facilitate switching on/off the onboard light source LS1, implement an example of causal synchronization logic SL. Switching events, in particular a switch off event at the marker device responds flexibly and dynamically to the light received from the endoscope light LS2. But Figs 3,4 are merely one embodiment for a causally linked synchronization logic SL. Other, switching event triggered logics that detect hard switching events are also envisaged herein in alternative embodiments. In these embodiments, interface and event handlers at the marker MD and/or endoscope ENDO capture "hard" switching events and then transmit a trigger signal to effect switching on or off the other switch of imager ENDO or marker MD to achieve a similar switching cycle pattern as in Fig 2. In these embodiments that are driven by hard switching events, the endoscope ENDO and the marker device MD may each include preferably a wireless transmitter/receiver arrangement that are coupled to the respective switches SW1, SW2. The switch event one in the endoscope or the marker device will then be transmitted, for instance from the marker device to the endoscope and to effect switching the endoscope on if the light source in the marker has been switched off or vice versa.

In the above described embodiments with causally linked synchronizer logic SL, it may not necessarily be required for the synchronizer logic to issue a specific switch off signal to cause the marker device light source LS1 to switch off so that the imager ENDO can operate in exploration mode, although this may still be implemented in some embodiments. Rather, as shown in the switch cycles of Fig 2, the marker light source LS1 may switch off automatically, either by expiry of a timing circuit or simply because of the power source being drained after sustaining a beacon pulse. The illumination source LS2 at the endoscope may then at one point be switched on again, e.g. at user request, and the marker power source PS1 is then recharged.

Turning now to the earlier mentioned non-causally linking synchronizer logic LS, in such embodiments the lights sources LS1, LS2 maybe switched autonomously, and yet the switching cycles as per Fig 2 or variants thereof can still be achieved. Such non-causally linking synchronizer logics SL may be implemented as two respective oscillating switching circuitries, one in the marker MD and one in the imager ENDO. The switching cycles are set up with pulse lengths for LS1, LS2 suitably timed, and out of phase. The user adjusts through a suitable interface at the imager and marker, frequency, phase and the respective lengths of switch off and switch on times so that the two cycles run autonomously out of phase similar to the diagrams of Fig 2. The marker may include a wireless set up functionality for the user to change its switching cycle post-implantation. There is no more a causal connection between the two switching logics and the system SYS can operate as described above. Two oscillator or multi-vibrators may be used, respectively integrated in the marker MD and the imager ENDO. Preferably, the switching off of the illumination light LS2 of the imager ENDO should be timed so as to allow the marker's MD power source PS1 to recharge if a light sensor LXS is used.

Referring now back to Fig 3 to describe the marker device MD more fully, the circuitry of the marker device is encapsulated or enclosed wholly or partly by a bio-compatible material CP. The material is preferably wholly translucent or may be only translucent at window portions W but is opaque elsewhere. In embodiment it is the whole of the encapsulation CP that is translucent. Translucent encapsulation ensures that more light can be sensed and released.

The translucent window portion(s) W are arranged where the light source and optionally the transducer PC are located. A single window portion W may be as shown in Figure 3 with suitable width to serve both, the light source and the transducer and/or light sensor. Alternatively, the transducer PC, if any, and the light sensor LXS and the light source may each have a separate discrete window portion (not shown).

Glass or other biocompatible materials are envisaged herein such as suitable polymers, or organic or inorganic translucent materials. Biocompatibility ensures in particular that toxic substances are not released into the surrounding tissue. The marker is not intended to stay long term in the patient but may be removed by a tool passed through the working channel of the endoscope ENDO at the conclusion of the intervention or in a follow-up intervention or may be removed together with the surrounding tissue during a surgical excision. Biocompatibility should be ensured at least for periods of days, preferably weeks or months because, due to pressure on health systems, it may well happen that considerable time may pass between implanting the marker and the concluding intervention.

The overall shape of the marker device can be any one of ball shape, ellipsoid shape but may also be arranged as a strip. Any other shapes are also envisaged. The overall dimensions may be in the millimeter or sub-millimeter range. Preferably the marker device is miniaturized so as to be able to be administered through a medical surgical needle. Examples include, biopsy needles of gauge 12-18, about 2-1.25mm in diameter, but thicker needles in the range of 3mm-5mm, such as for ablation purposes, may also be envisaged.

The overall shape of the encapsulation CP is generally convex so that it can be easily administered through the needle as mentioned. Alternatively, however, the encapsulation CP may not necessarily be convex, in particular, it may include protruding structures to form hooks or other anchorages so as to assist in affixing the marker to the intended tissue type or organ. The encapsulation CP may be monolithic or may be assembled from components or parts.

With continued reference to Figure 3, the marker device MD may optionally include further circuitry or components that allow the marker device to collect measurement data of physical, chemical or physiological quantities. One or more sensors or probes S may be so arranged. The measurement values may be stored in an onboard memory and can then be evaluated once the marker device is removed. Alternatively, there is a dedicated transmitter that transmits to the outside measurement data which can be evaluated by a receiver, such as a computing device. In one embodiment there is no dedicated transmitter, but a modulator MOD that modulates the measurement values onto the light emitted by light source LS1. The modulation may occur during beacon mode, or may occur instead in a separate operational phase when light source LS1 is solely activated to transmit the measured values. Modulation may be through frequency modulation or intensity modulation or a combination.

The transducer TD, although preferred, is optional, and the marker MD may be powered by an on-board battery or other energy source, without the transducer, especially when used with the non-causally linking synchronizer logic SL. Preferably, however a transducer is used in the marker MD to recharge the marker light source LS1 during exploration mode, suing the light from the imager ENDO light source LS2. The transducer TD may be used in the causal or non-causal embodiments of the synchronizer logic SL.

Turning now in more detail to the operation of the image processor IP, this may extract image information from the dark or beacon images. In particular, the location of the isolated signal, the bright spot, may be determined by thresholding or segmentation. The location so determined may then be suitably indicated in the follow up one or more exploratory images. A suitable graphical symbol such as hair-cross or other may be overlaid in the exploratory images at the corresponding in-image location in the one or more subsequent exploratory images to provide a clue to the user of the location of the marker device. Alternatively, the whole beacon image is overlaid onto the exploratory image. "Dark" areas of the beacon image are rendered completely transparent, whilst light areas that represent the marker light signal, are rendered opaque or only partially transparent. The marker location, and the associated graphical symbol, is dynamically updated when a new beacon image is acquired, and so on. In other embodiments, the bright spot is segmented, and it is the segmentation that is then overlaid.

If the movement of the endoscope in between two frames, in particular between acquisition of the beacon image and an exploratory frame, is zero or negligible, the two images are naturally registered spatially with one another. In other words, the same in-image co-ordinates in the two images correspond to the same spatial position. In this case, no dedicated registration operation is required, and the marker position that can be readily indicated in the exploratory image based on the signal in the earlier or later beacon image. Otherwise, if there is non-negligible motion between the frames and hence no direct native registration may be assumed, a registration functionality of the image processor may be employed to attempt spatially registering one of the earlier or later beacon frames and a follow-up or earlier exploratory frame. In situations in which only exploratory images can registered with each other, a beacon image is then registered on temporally neighbored exploratory image(s), which are then in turn registered to later acquired exploratory images, so as to ensure that also those later exploratory images may be faithfully registered with the beacon image.

Morphing algorithms, optical flow methods or others may be used to effect the registration. The image registration processing may be beneficial in particular in situations where the time to recharge the marker power source PS 1 is relatively long, and where the period between subsequent LS1 -pulses might lead to spatial a mismatch between the beacon image and the current exploratory image. The proposed image registration allows to address this.

In embodiments, before attempting to locate the marker position in the beacon image, a contrast enhancer CE may be used to contrast enhance the image and to then attempt the identification. A color change may also be attempted instead or in addition to contrast enhancement.

In order to capture better beacon images of enhanced quality, the image apparatus ENDO may include a filter component FC. The filter component has a frequency response function that corresponds to the bandwidth of the light source LS 1 of the marker. The light from the marker device MD captured by the endoscope ENDO during beacon mode is then first filtered by the component FC, and it is the so filtered image that is then processed as described above.

In sum, as per the above embodiments, the image processor is configured to combine at least a part of the beacon image with the exploratory images to form a combined image that may include additional graphical information to indicate the location of the marker device to better assist the user in navigating towards the device.

However other visualization options of the marker location are also envisaged. For instance, in embodiments it is the beacon image itself that is displayed, for instance by interleaving the beacon images into the stream of exploratory images. The so interleaved beacon images may be displayed for a certain amount of time. The time may be set by the user. The time may be short, such as a fraction of a second, or in the order of one or more seconds.

Alternatively, the beacon images are displayed as a separate stream alongside the stream of exploratory images, either in different, respective, screen portions of a single display device, or on two display devices, respectively.

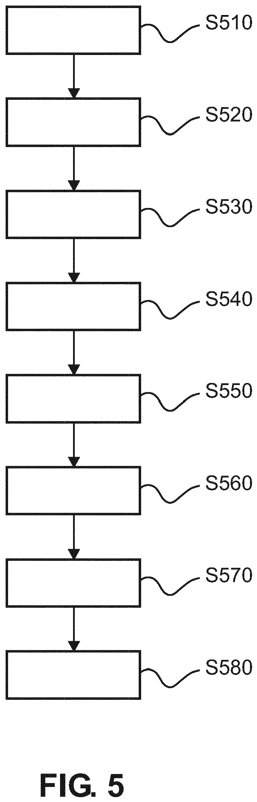

Reference is now made to Figure 5 which slows a flow chart of a method for marker based navigation. The method can be used to implement the system as described above in Figures 1-4 but the following steps of the method may be understood as a teaching in its own right.

At step S510, the light source of the implanted device MD is switched off.

In step S520, the light source LS2, the illumination source, at the medical imaging apparatus such as an endoscope is switched on.

The switching on of the light source may occur in a response to a signal received in connection with the switching off of the light source of the implanted device. The order of the two steps S510 and S520 may be reversed. Now that the second light source of medical imaging device that illuminates the field of view of the medical device is on, whilst the light source at the implanted device is switched off, one (or more) image is captured with the imaging apparatus at step S530. This image may be referred to as an exploratory image and constitutes an image suitably exposed thanks to the second light source.

Once one or more of such exploratory images have been captured, the light source at the medical device is switched off at step S540. The light source at the marker device is switched on at step S550. Again, the steps S540 and S550 may be reversed.

In embodiments, the switching on in step S540 of the marker light source responds to the switching off in step S550 of the illumination source at the imager. Now that the second light source at the imager is off and the light source at the implanted device is switched on, a second (one or more) image is captured by the medical imager at step S560. This second image may be referred to as the beacon image. This image will capture an isolated light signal, a light spot, from the marker device to so indicate the location of the implanted marker device (and hence of the lesion) although the marker it may not necessarily be in a line of sight relative to the medical imaging apparatus. Because the beacon image is now underexposed and may not reveal the surroundings as the illumination source is off, the location of the marker is precisely indicated thanks to the isolated light signal from the marker device as recorded in the beacon image.

At an optional step S570, at least a part of the image information in the dark image and at least a part of an exploratory image may be combined.

In particular, the light spot representing the light from the marker device may be identified in the beacon image through segmentation, signal thresholding or other image processing. The in-image position of the so identified light spot may be indicated in the exploratory image, to so reveal to the user the location of the, possibly occluded, marker and hence of the lesion. The location may be indicated by overlaying a visual marker at the corresponding location in the exploratory image.

At step S580 the combined image may be displayed on a display device as part of a video feed or a still image. The new imagery can be used to support an intervention and to reliably guide the user to the marker and hence the lesion marked by the marker.

The proposed method as such results in two streams, one for the beacon images and one for the exploratory images. The image streams may be interleaved, so as to generate a stream of exploratory images runs, occasionally interrupted by one or more beacon images, when a beacon image becomes available. In embodiments there may not necessarily be a strict, regular alteration between beacon image and exploratory image.

Steps S540, S550 may be performed before the step S520, S530.

In embodiments there may be causal link between steps S540 and S550 or between steps S520 and S530.

The above steps may be repeated, to obtain new pairs of exploratory and beacon images.

In particular, the switching on of the marker light may be caused by the switching off of the illumination source at the imager. This will constitute a causal link synchronization but this is not necessarily required in all embodiments, as non-causal synchronization is also envisaged. In non-causal synchronization the two light sources may be pre-set to pulse in frequencies suitably adjusted out of phase with pulse lengths suitably chosen as explained above at Fig 2.

For best results, it may be advisable to darken the operating theatre to reduce ambient light that may trigger undesirable switching operations. In embodiments, a calibration procedure may be advisable to calibrate the marker sensor to the light source of the imaging apparatus. The light emitted by the marker and/or the illumination source may include visible light, but other frequencies such as IR or NIR are also envisaged.

Preferably, the marker as used in the proposed method is powered by light, in particular by light of the imager's illumination source.

The capturing of the beacon and exploratory image at steps S530, S560 may include using a lensed eye piece or view finder such as may be used in an endoscope. The location of the marker as identified may be overlaid as a visual indication in the field of view of the eye piece or view finder.

The components of the image processing system IP, the modulation module MD and or part or all of the synchronizer logic SL may be implemented as software modules or routines run on a computing unit PU such as a workstation associated with the imager ENDO. The image processing system IP, the modulation module MD and/or a part or all of the synchronizer logic SL may be arranged in a distributed architecture and connected in a suitable communication network. The image processing system IP, the modulation module MD and/or a part or all of the synchronizer logic SL may be arranged in the marker device or in the imager ENDO, as a suitably programmed microprocessor or microcontroller, such as an FPGA (field-programmable-gate-array) or as hardwired IC chip such as an ASIC, system-on-a-chip (SOC), and combinations thereof.

The switching on and off of one or both of the light sources LS1, LS2 may be done by (re-)connection or disconnection, respectively, from the respective power source. Alternatively, the light may be blocked /released by a shutter mechanism or filter element.

In all of the above embodiments, instead of switching off one or both of the lights sources LS1, LS2, the light intensities may be merely reduced to a non-zero intensity level by a dimmer functionality or by insertion of a filter element or other. In particular, the light source LS2 at the imager may be so dimmed when the light source LS1 of the marker is on. The marker is preferably not merely dimmed, but switched off when the light source of the imager is switched to the higher intensity. In other embodiments, it is the light source at the marker that is dimmed whilst the light source of the imager is illuminating at a higher intensity level. In a further embodiment, each light source is merely dimmed whilst the other light source illuminates at a higher intensity, and there is no complete switch off at either light source.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. System (SYS) for marker-based navigation, comprising:
an implantable marker device (MD) having a light source, switchable into at least two intensity states, a low intensity state and a high intensity state;
a synchronizer logic (SL); and
an imaging apparatus (ENDO) with a second light source (LS2) to provide incident light, the said second light source switchable into at least two intensity states, a low intensity state and a high intensity state;
wherein the light source (LS1) of the marker device (MD) and the second light source (LS2) are operable in synchrony by the synchronizer logic (SL) controlling the switching of the two light sources (LS1, LS2), the system thereby operable in two modes, in a beacon mode and in an exploration mode, so that, in exploration mode, the light source (LS1) is switched into low intensity state and the second light source (LS2) is switched into high intensity state, and so that in beacon mode, the light source (LS1) is switched into high intensity state and the second light source (LS2) is switched into low intensity state, the imaging apparatus (ENDO) controlled by the synchronizer logic (SL), or by a user, to capture at least one respective image in the two modes.

2. System of claim 1, including an image processor (IP) configured to combine image information from the at least two images to form a combined image.

3. System of claim 1 or 2, the image processor (IP) comprising a contrast enhancer (CE) to enhance contrast in the one or more images acquired in beacon mode.

4. System of any one of the preceding claims, the imaging apparatus (ENDO) including a light filter component (FC) having its frequency response adjusted to a bandwidth of the light emittable by the light source (LS1) of the marker device (MD).

5. System of any one of the preceding claims, the image processor (IP) configured to spatially register the image captured in beacon mode and the image captured in exploration mode.

6. System of any one of the preceding claims, wherein the synchronizer logic (SL) comprises a light sensitive sensor (LXS), the light sensitive sensor operable to effect the switching into the two intensities of the first or the second light source, in dependence on incident light received from second or the first light source, respectively.

7. ystem of claim 6, wherein the light sensitive sensor (LXS) is configured to facilitate i) the first light source to switch into high intensity state in response to the sensor sensing a light intensity below an intensity threshold and/or ii) the first light source to switch into low intensity state in response to the sensor sensing a light intensity above the threshold.

8. System of claim 7, wherein the light intensity sensed at the sensor (LXS) is caused by the incident light from the second light source (LS2).

9. An implantable marker device (MD) for navigation support, comprising:
a light source (LS1) configured to emit light;
a power source (PS), configured to power the light source;
a light sensor (LXS) configured to sense ambient light; and
a switch (SW) configured to switch the light source into at least two intensity states, a low intensity state and a high intensity state, in dependence on a light exposure at the light sensor (LXS).

10. Device of claim 9, wherein the switch (SW) is configured to switch the light source (LS1) into the high intensity state when an intensity of the incident light drops under an intensity threshold.

11. A medical imaging device (ENDO), comprising an image sensor (LD) to capture an image and a light source (LS2) to provide ambient light for the light sensor (LD), and a synchronizer logic (SL) operable to cause the light source (LS2) being switched into at least two intensity states, a low intensity state and a high intensity state, whilst capturing images, the said switching on and off being set at a preset frequency or wherein the switching between the two intensities is in response to an external switching signal received by the synchronizer logic (SL) through an interface (IFE) of the device (ENDO).

12. Method for marker-based navigation, comprising the steps of:
capturing (S530) a first image with a medical imaging apparatus (ENDO), whilst a light source (LS1) of an implanted marker device is in a low intensity state and a second light source (LS2) of the medical imaging apparatus (ENDO) is in a high intensity state, higher than the low intensity state of the marker; and
capturing (S560) a second image with the medical imaging apparatus (ENDO), whilst the light source (LS1) of the implanted marker device is in a high intensity state and the second light source (LS2) of the medical imaging apparatus (ENDO) is in a low intensity state, lower than the high intensity state of the marker.

13. Method of claim 12, comprising: combining (S570) the two images and, optionally, displaying (S580) the combined image on a display device (DD), and or displaying the two images.

14. A computer program element, which, when being executed by at least one processing unit (SL, PU), is adapted to cause the processing unit (SL, PU) to perform one or more steps of the method as per claim 12 or 13.

15. A computer readable medium having stored thereon the program element of claim 14.
